## Europäisches Patentamt
## European Patent Office
## Office européen des brevets

(11) Publication number: **0 169 700**
**B1**

# EUROPEAN PATENT SPECIFICATION

(45) Date of publication of patent specification: **16.05.90**

(21) Application number: **85305051.6**

(22) Date of filing: **15.07.85**

(51) Int. Cl.⁵: **A 61 K 31/545**

(54) Improved pharmaceutical formulations for ceftazidime.

(30) Priority: **23.07.84 US 633739**

(43) Date of publication of application:
**29.01.86 Bulletin 86/05**

(45) Publication of the grant of the patent:
**16.05.90 Bulletin 90/20**

(84) Designated Contracting States:
**BE CH DE FR GB IT LI LU NL SE**

(56) References cited:
**FR-A-2 466 467**

(73) Proprietor: **ELI LILLY AND COMPANY**
**Lilly Corporate Center**
**Indianapolis Indiana 46285 (US)**

(72) Inventor: **Fites, Alan Lee**
**4003 South Drive Cedar Hills**
**Greenwood Indiana 46142 (US)**
Inventor: **Pikal, Michael Jon**
**540 Leisure Lane**
**Greenwood Indiana 46142 (US)**

(74) Representative: **Hudson, Christopher Mark et al**
**Erl Wood Manor**
**Windlesham Surrey GU20 6PH (GB)**

Courier Press, Leamington Spa, England.

## Description

This invention relates to pharmaceutical formulations. In particular, it relates to pharmaceutical formulations of the semi-synthetic cephalosporin antibiotic known as ceftazidime.

The antibiotic ceftazidime is described by O. Callahan, el al., in U.S. Patent No. 4,258,041 and is chemically named (6R, 7R) - 7 - [(Z) - 2 - (2 - aminothiazol - 4 - yl) - 2 - (2 - carboxyprop - 2 - oxyimino)acetamido] - 3 - (1 - pyridiniummethyl) - 3 - cephem - 4 - carboxylate. Ceftazidime has been obtained as a crystalline pentahydrate, a useful form for pharmaceutical formulations of the antibiotic. Ceftazidime pentahydrate is described by Brodie, et al., in U.S. Patent No. 4,329,453. Previously known formulations of ceftazidime pentahydrate degrade, particularly when stressed at higher than room temperatures. This degradation can lead to the development of high molecular weight polymer in the formulation. This degradation, leading to polymers having a molecular weight of about 10,000 or greater, has been observed to impart substantial toxicity to the formulation. Further such degradation diminishes the pharmaceutical elegance of the formulations and is to be avoided for that reason alone.

The formulations of ceftazidime pentahydrate provided by this invention suppress the formation of the toxic polymers having a molecular weight greater than about 10,000, even upon stress of the formulation, and, thus, provide a more pharmaceutically elegant product for administration.

In particular, this invention provides a pharmaceutical formulation comprising crystalline ceftazidime pentahydrate, a pharmaceutically-acceptable base and amorphous lactose. The incorporation of amorphous lactose in formulations of ceftazidime pentahydrate suppresses the formation of polymeric substances, including polymers having a molecular weight of 10,000 or higher. The formulations provided herein are in solid form and are suitable for parenteral administration in antibiotic therapy upon dilution with a physiological diluent.

During accelerated stability studies of the antibiotic ceftazidime pentahydrate, degradation of the antibiotic along with formation of polymeric substances of unknown composition occurred. Among the polymeric substances produced are those having a molecular weight of 10,000 or greater. Materials containing such polymeric degradation products were found to exhibit extreme toxicity in rabbits. Many preparations of ceftazidime pentahydrate maintained on long-term stability studies at ordinary conditions of temperature and humidity also exhibited degradation leading to polymer formation to a greater or lesser extent than occurred with the accelerated studies.

According to the present invention, formulating amorphous lactose with crystalline ceftazidime pentahydrate suppresses and retards the degradation of the antibiotic and, in particular, degradation leading to polymeric substances having a molecular weight of 10,000 or greater. Thus, this invention provides a pharmaceutical formulation comprising ceftazidime pentahydrate, a pharmaceutically acceptable base, and amorphous lactose.

Lactose occurs in both an α- and a β-form, either of which may be employed in the formulations of this invention as long as the lactose is amorphous. Alpha-lactose is commonly available and used in pharmaceutical formulations as the crystalline monohydrate. The crystalline monohydrate of α-lactose is unsuitable in the formulations of this invention, although it can be present in minor amounts in the amorphous form employed in the formulations of this invention. Preferably, the lactose, be it the β-form or the α-form, is at least 90% in the amorphous form.

Since it appears that unbound water may be associated with the instability of ceftazidime, the amorphous lactose employed in the formulations desirably has a water content below 5% by weight, such as below 3% by weight, and preferably no more than 1% or 2% by weight. It appears that crystalline lactose monohydrate is ineffective in stabilizing ceftazidime since it has little tendency to absorb moisture as does the amorphous form.

The formulations of this invention comprise amorphous lactose in an amount corresponding to between 10% and 50% of the weight of ceftazidime pentahydrate employed in the formulation.

A pharmaceutically-acceptable base is included in the formulation to provide sufficient base to form a water-soluble salt of ceftazidime. A "pharmaceutically-acceptable base" refers to those bases used in forming salts of ceftazidime useful for the chemotherapy of warm-blooded animals. Suitable pharmaceutically-acceptable bases include, for example, the alkali metal carbonates, such as sodium carbonate, and potassium carbonate; the alkali metal bicarbonates, such as sodium bicarbonate and potassium bicarbonate; and other suitable bases, such as ammonium carbonate. The amount of base employed in the composition is an amount sufficient to form the salt form of the amount of ceftazidime present.

The ceftazidime formulations provided by this invention are prepared in a conventional manner. For example, the ceftazidime pentahydrate may be mixed in a blender with the base and the amorphous lactose, and then the mix may be filled into vials. Alternatively, the ceftazidime pentahydrate may be filled into a suitable parenteral vial, followed by addition of the base and lactose without prior blending. Preferably the ceftazidime pentahydrate is mixed intimately with the pharmaceutically-acceptable base, for example, sodium carbonate, and then the amorphous lactose is added to the blend. The lactose need not be thoroughly blended with the ceftazidime pentahydrate and base mix to effectively stabilize the formulation.

Amorphous lactose having a desired water content of less than 3% may be prepared in a number of ways, for example, by freeze drying an aqueous solution of lactose or by spray drying. Amorphous lactose obtained by freeze drying is preferred. Freeze-dried amorphous lactose is obtained, for example, by first preparing a solution of between 5 and 15% of lactose in water. The solution is then frozen at a temperature of about −40°C and dried under a vacuum while the temperature of the frozen solution is maintained at a temperature between about −30 and about −38°C. When ice sublimation is completed, the amorphous lactose obtained is dried further at a higher temperature, for example, between about 40 and about 80°C. This process provides amorphous lactose having a water content of 1% to 2%. The bulk-powder density of the amorphous lactose prepared by freeze drying varies with the concentration of the aqueous lactose solution. The more concentrated lactose solutions provide amorphous lactose with higher bulk-powder density. Accordingly, a concentrated solution of lactose of about 15% by weight provides amorphous form of lactose with the highest bulk-powder density.

The pharmaceutical formulations of this invention preferably contain between 10% and 15% of dry powdered sodium carbonate and between 10% and 50% of amorphous lactose per weight of ceftazidime activity. One gram of ceftazidime activity corresponds to more than one gram of ceftazidime pentahydrate owing to the weight of water of hydration. This invention further provides antibiotic formulations of ceftazidime in unit dosage form. One such formulation comprises 1 g of ceftazidime activity, 116 mg of sodium carbonate, and 250 mg of amorphous lactose. Another dosage unit formulation comprises 1 g of ceftazidime activity in the form of ceftazidime pentahydrate, 116 mg of sodium carbonate, and 500 mg of amorphous lactose. A larger dosage unit formulation provided herein comprises 2 g of ceftazidime, 232 mg of sodium carbonate, and 500 mg of amorphous lactose.

The formulations of this invention are useful for administration by the parenteral route, for example, i.v. or i.m. For such administration they can be incorporated into rubber-stoppered glass vials or into plastic bags suitable for administration by the i.v. drip method. The formulations can be incorporated into glass vials and plastic bags by conventional filling procedures. Because of the ability of amorphous lactose to absorb moisture, the filling operation should be carried out in a dry atmosphere, for example, in an atmosphere having a relative humidity of 20 to 30% at room temperature.

The pharmaceutical formulations of this invention may be stored at room temperatures for extended periods of time without the formation of degradation products, including polymeric substances having a molecular weight of 10,000 or greater. The amorphous lactose incorporated in the formulations of this invention suppresses the degradation of ceftazidime, including the development of the undesirable polymeric substances having a molecular weight of 10,000 or greater. For example, formulations of the invention comprising ceftazidime pentahydrate, dry powdered sodium carbonate, and 25% by weight of amorphous lactose or 50% by weight of amorphous lactose inhibited the development of the high molecular weight polymeric substances in accelerated stability studies carried out at 60°C for three days. The control lot of ceftazidime plus sodium carbonate carried out in the same test showed the presence of 0.87% of polymeric substances having a molecular weight of 10,000 or greater. Formulations of the invention comprising, respectively, 25% and 50% by weight of amorphous lactose per weight of ceftazidime activity, assayed for only .12% polymeric substances having a molecular weight of 10,000 or greater.

The assay employed in determining the amount of high molecular weight polymer in the ceftazidime formulations employs gel chromatography (HPLC) using a gel having an exclusion limit of about 10,000. A suitable gel for use in the assay is Fractogel® TSK HW-40 (Merck). The assay is carried out in a glass HPLC column measuring 50 cm in length by 0.9 cm i.d. packed with a gel in potassium phosphate solution. A 10 mg-sample of the ceftazidime formulation is dissolved in 5 ml of pH 7 phosphate buffer. The sample solution, 100 microliters, is injected and the column is run at a flow rate of 1 ml/min. The column is run at room temperatures, and a photometric detector suitable for use in HPLC applications is run at 0.1 AUFS at 210 nm.

The following non-limiting examples further illustrate the present invention.

Example 1
Preparation of amorphous lactose via freeze drying

A solution of lactose monohydrate in water having a concentration of between 5% and 15% w/w is poured into stainless steel pans prechilled to about −35°C. The freeze dryer shelf temperature is maintained at −40°C until the solution is frozen. The drying chamber is evacuated and primary drying is conducted at a temperature below −31.5°C (pan contents). The temperature of the pan contents (frozen solution) is maintained at a temperature of between −33°C and −38°C by control of the shelf temperature at between −15°C and −18°C and a chamber pressure of between about 60 microns and 70 microns. When ice sublimation is complete as indicated by the temperature readings of the pan contents, secondary drying is conducted at a shelf temperature of about 25°C for 15 h and at about 40°C for 3 h. The amorphous lactose obtained has a water content of between 1% and 2%.

The amorphous lactose is stored in water vapor tight containers or in a dry atmosphere to prevent absorption of moisture.

Example 2

Crystalline ceftazidime pentahydrate is blended with 10% by weight of dry, powdered sodium carbonate and filled into glass vials to provide 1 gram of ceftazidime activity and 116 mg of sodium carbonate per vial. To each vial is then added 250 mg of amorphous lactose, and the vials are then closed with a butyl rubber stopper.

Example 3

Ceftazidime pentahydrate is blended with 10% by weight of dry powered sodium carbonate and 50% by weight of amorphous lactose prepared by the freeze drying method described by Example 1. The blended mixture is then filled into vials each containing 1 g of ceftazidime activity, 116 mg of dry powdered sodium carbonate, and 500 mg of amorphous lactose.

**Claims**

1. A pharmaceutical formulation comprising (6R, 7R) - 7 - [(Z) - 2 - (2 - aminothiazol - 4 - yl) - 2 - (2 - carboxyprop - 2 - oxyimino)acetamido] - 3 - (1 - pyridiniummethyl) - 3 - cephem - 4 - carboxylate pentahydrate, a pharmaceutically acceptable base, and amorphous lactose.

2. A formulation as claimed in claim 1 in which the pharmaceutically-acceptable base is sodium carbonate.

3. A formulation as claimed in claim 1 or 2 in which the amorphous lactose has a water content of less than 3%.

4. A formulation as claimed in any one of claims 1 to 3 comprising ceftazidime pentahydrate, between 10% and 15% by weight of ceftazidime activity of dry powdered sodium carbonate, and between 10% and 50% by weight of ceftazidime activity of amorphous lactose.

5. A formulation as claimed in claim 4 in which amorphous lactose is present in an amount corresponding to 25% by weight of ceftazidime activity.

6. A formulation as claimed in claim 4 in which the amorphous lactose is present in an amount corresponding to 50% by weight of ceftazidime activity.

7. A pharmaceutical formulation as claimed in claim 4 in unit dosage form comprising 1 g of ceftazidime activity, 116 mg of dry powdered sodium carbonate, and 250 mg of amorphous lactose.

8. A pharmaceutical formulation as claimed in claim 4 in unit dosage form comprising 1 g of ceftazidime activity, 116 mg of dry powdered sodium carbonate, and 500 mg of amorphous lactose.

9. A pharmaceutical formulation as claimed in claim 4 in unit dosage form comprising 2 g of ceftazidime activity, 232 mg of sodium carbonate, and 500 mg of amorphous lactose.

10. A pharmaceutical formulation as claimed in claim 1 for use in the treatment of an antibiotic-susceptible diseases in a warm-blooded animal.

**Patentansprüche**

1. Pharmazeutische Formulierung, dadurch gekennzeichnet, daß sie (6R, 7R) - 7 - [(Z) - 2 - (2 - Aminothiazol - 4 - yl) - 2 - (2 - carboxyprop - 2 - oxyimino)acetamido] - 3 - (1 - pyridiniummethyl) - 3 - cephem - 4 - carboxylatpentahydrat, eine pharmazeutisch annehmbare Base un amorphe Lactose enthält.

2. Formulierung nach Anspruch 1, dadurch gekennzeichnet, daß die pharmazeutisch annehmbare Base Natriumcarbonat ist.

3. Formulierung nach Anspruch 1 oder 2, dadurch gekennzeichnet, daß die amorphe Lactose einen Wassergehalt von weniger als 3% hat.

4. Formulierung nach einem der Ansprüche 1 bis 3, dadurch gekennzeichnet, daß sie Ceftazidimpentahydrat, zwischen 10 und 15 Gewichtsprozent der Ceftazidimaktivität an trockenem Natriumcarbonat und zwischen 10 und 50 Gewichtsprozent der Ceftazidimaktivität an amorpher Lactose enthält.

5. Formulierung nach Anspruch 4, dadurch gekennzeichnet, daß die amorphe Lactose in einer Menge vorhanden ist, die 25 Gewichtsprozent der Ceftazidimaktivität entspricht.

6. Formulierung nach Anspruch 4, dadurch gekennzeichnet, daß die amorphe Lactose in einer Menge vorhanden ist, die 50 Gewichtsprozent der Ceftazidimaktivität entspricht.

7. Pharmazeutische Formulierung nach Anspruch 4 in Einheitsdosierungsform, dadurch gekennzeichnet, daß sie 1 g Ceftazidimaktivität, 116 mg trockenes, pulverförmiges Natriumcarbonat und 250 mg amorphe Lactose enthält.

8. Pharmazeutische Formulierung nach Anspruch 4 in Einheitsdosierungsform, dadurch gekennzeichnet, daß sie 1 g Ceftazidimaktivität, 116 mg trockenes, pulverförmiges Natriumcarbonat und 500 mg amorphe Lactose enthält.

9. Pharmazeutische Formulierung nach Anspruch 4 in Einheitsdosierungsform, dadurch gekennzeichnet, daß sie 2 g Ceftazidimaktivität, 232 mg Natriumcarbonat und 500 mg amorphe Lactose enthält.

10. Pharmazeutische Formulierung nach Anspruch 1 zur Verwendung bei der Behandlung antibiotikumsuszeptibler Krankheiten bei einem warmblütiger Tier.

**Revendications**

1. Formulation pharmaceutique comprenant le pentahydrate de (6R, 7R) - 7 - [(Z) - 2 - (2 - aminothiazol - 4 - yl) - 2 - (2 - carboxyprop - 2 - oxyimino) - acétamido] - 3 - (1 - pyridinium - méthyl) - 3 - céphem - 4 - carboxylate, une base pharmaceutiquement acceptable, ainsi que du lactose amorphe.

2. Formulation selon la revendication 1, dans laquelle la base pharmaceutiquement acceptable est le carbonate de sodium.

3. Formulation selon la revendication 1 ou 2, dans laquelle le lactose amorphe a une teneur en eau inférieure à 3%.

4. Formulation selon l'une quelconque des revendications 1 à 3, comprenant du pentahydrate de ceftazidime, entre 10 et 15% en poids (calculé sur l'activité de ceftazidime) de carbonate de sodium sec en poudre, ainsi qu'entre 10 et 50% poids, calculé sur l'activité de ceftazidime, de lactose amorphe.

5. Formulation selon la revendication 4, dans laquelle le lactose amorphe est présent en une quantité correspondant à 25% en poids d'activité de ceftazidime.

6. Formulation selon la revendication 4, dans laquelle le lactose amorphe est présent en une quantité correspondant à 50% en poids d'activité de ceftazidime.

7. Formulation pharmaceutique selon la revendication 4, sous une forme posologique unitaire comprenant 1 g d'activité de ceftazidime, 116 mg de carbonate de sodium sec en poudre et 250 mg de lactose amorphe.

8. Formulation pharmaceutique selon la revendication 4, sous une forme posologique unitaire comprenant 1 g d'activité de ceftazidime, 116 mg de carbonate de sodium sec en poudre et 500 mg de lactose amorphe.

9. Formulation pharmaceutique selon la revendication 4, sous une forme posologique unitaire comprenant 2 g d'activité de ceftazidime, 232 mg de carbonate de sodium et 500 mg de lactose amorphe.

10. Formulation pharmaceutique selon la revendication 1, en vue de l'utiliser pour le traitement des maladies susceptibles aux antibiotiques chez un animal à sang chaud.